# EUROPEAN PATENT APPLICATION

(11) **EP 4 830 647 A2**
(43) Date of publication of application: **28.10.2026**
(21) Application number: 26204978.6
(22) Date of filing: 15.11.2019
(51) Int. Cl.: A61B 17/122

(54) **SURGICAL CLIP**

(30) Priority: 16.11.2018 US 201862768671 P
(62) Document number(s) of the earlier application(s) in accordance with Art. 76 EPC: 19884321.1 / 3 880 085
(71) Applicant: Teleflex Medical Incorporated, Morrisville, North Carolina 27560 (US)
(72) Inventor: FOSHEE, David Lee, Apex, North Carolina 27539 (US); CASTRO, Salvatore, Raleigh, North Carolina 27603 (US)
(74) Representative: Jacob, Reuben Ellis

(57) **Abstract**

A surgical clip may include a first leg member having a first inner surface with a concave curvature, a second leg member having second inner surface with a convex curvature, and a hinge member pivotally coupling the first leg member and the second leg member. The first leg member may have a first thickness in a compression direction, a first width in a lateral direction, and a first length in a longitudinal direction. The second leg member may have a second thickness in the compression direction, a second width in the lateral direction, and a first length in the longitudinal direction. The first width may be greater than the first thickness along at least half of the first length, and/or the second width may be greater than the second thickness along gat least half of the second length.

## Description

### Priority

The present application claims priority to U.S. Provisional Pat. App. No. 62/768,671 (filed on November 16, 2018), the entire disclosure of which is incorporated herein by reference.

### Technical Field

The present invention relates generally to medical devices, and more particularly, to surgical clips for ligation of tissue.

### Background

Ligation of tissue (*e.g.*, blood vessels, lymph nodes, nerves, cystic ducts, and cardiac tissue) is a common practice for many surgical procedures. This can be performed by closing the vessel with a surgical clip or by suturing the vessel with the surgical thread. The use of surgical thread requires complex manipulations of a needle and surgical thread to form knots required to secure the vessel. Such complex manipulations are time consuming and difficult to perform, particularly in endoscopic surgical procedures characterized by limited space and/or visibility. In contrast, surgical clips are relatively quick and easy to apply. Accordingly, the use of surgical clips in endoscopic and open surgical procedures has grown dramatically.

### Summary

The present inventors recognize that there is a need to improve one or more features of the surgical clips, such as the tissue-retaining capacity of the surgical. The disclosed devices and methods are directed to mitigating or overcoming one or more of the problems set forth above and/or other problems in the prior art.

An aspect of the present invention is directed to a surgical clip. The surgical clip may include a first leg member having a first inner surface with a concave curvature, a second inner surface with a convex curvature, and a hinge member pivotally coupling the first leg member and the second leg member. The first leg member may have a first thickness in a compression direction, a first width in a lateral direction, and a first length in a longitudinal direction. The second leg member may have a second thickness in the compression direction, a second width in the lateral direction, and a first length in the longitudinal direction. The first width may be greater than the first thickness along greater than half of the first length, and/or the second width may be greater than the second thickness along greater than half of the second length.

In some embodiments, the first width is at least 1.5 times greater than the first thickness and/or the second width is at least 1.5 times greater than the second thickness. In some embodiments, the first width is about 2 times greater than the first thickness and/or the second width is about 2 times greater than the second thickness. In some embodiments, the first width is greater than the first thickness along at least about two-thirds of the first length, and/or the second width is greater than the second thickness along at least about two-thirds of the second length. In some embodiments, the surgical clip further includes at least one first boss member on the first leg member; and at least one second boss member on the second leg member, wherein the first width is substantially the same as a width of the surgical clip at the at least one first boss member, and/or the second width is substantially the same as a width of the surgical clip at the at least one second boss member. In some embodiments, the first width is substantially the same as a width of the hinge member, and/or the second width is substantially the same as the width of the hinge member. In some embodiments, the first leg member includes at least one first wing member extending laterally of the first inner surface to define the first width, and/or the second leg member includes at least one second wing member extending laterally of the second inner surface to define the second width. In some embodiments, the at least one first wing member includes a pair of first wing members, and/or the at least one second wing member includes a pair of second wing members. In some embodiments, a distal portion of the at least one first wing member is spaced proximally from a distal end of the first leg member and/or a distal portion of the at least one second wing member is spaced proximally from a distal end of the second leg member. In some embodiments, the at least one first wing member has a thickness less than the first thickness and/or the at least one second wing member has a thickness less than the second thickness. In some embodiments, the at least one first wing member and/or the at least one second wing member includes an inner surface that is beveled. In some embodiments, the at least one first wing member includes an outer surface that is continuous with an outer surface of the first leg member and/or the at least one second wing member includes an outer surface that is continuous with an outer surface of the second leg member. In some embodiments, each of the at least one first wing member has a width no great than about a quarter of the first width and/or each of the at least one second wing member has a width no greater than about a quarter of the second width. In some embodiments, the at least one first wing member and/or the at least one second wing member includes a side surface that is substantially flat. In some embodiments the surgical clip further includes a hook member on a distal portion of the first leg member; and a tip member on a distal portion of the second leg member, wherein the hook member is configured to receive the tip member to retain the surgical clip in a closed configuration. In some embodiments, the surgical clip includes first and second rows of teeth extending from one of the first inner surface and the second inner surface; first and second channels on another of the first inner surface and the second inner surface; wherein, in a closed configuration, the first channel is configured to receive the first row of teeth, and the second channel is configured to receive the second row of teeth. In some embodiments, the surgical clip, further includes a first proximal extension extending from the first leg member toward the hinge member; and a second proximal extension extending from the second leg member toward the hinge member. In some embodiments, the first proximal extension comprises an inner surface having a convex curvature, and the second proximal extension comprises an inner surface having a convex curvature. In some embodiments, the surgical clip further includes a first inner member at a proximal portion of the first leg member; and a first aperture at a proximal portion of the second leg member, wherein the first aperture is configured to receive the first inner member in a closed configuration to resist the surgical clip from inverting and/or rotating, and the first inner member does not latch and/or interlock with the first aperture. In some embodiments, the surgical clip further includes a second inner member at a proximal portion of the second leg member; and a second aperture at a proximal portion of the first leg member, wherein the second aperture is configured to receive the second inner member in a closed configuration to resist the surgical clip from inverting and/or rotating, and the second member does not latch and/or interlock with the second aperture.

### Brief Description of the Drawings

In order that the invention may be readily understood, aspects of this invention are illustrated by way of examples in the accompanying drawings.
FIG. 1 illustrates a side view of a first exemplary embodiment of a surgical clip of the present invention.
FIG. 2 illustrates a side view of a compressed or closed configuration of the first exemplary embodiment of the surgical clip of FIG. 1.
FIG. 3 illustrates a perspective view of the first exemplary embodiment of the surgical clip of FIG. 1 and 2.
FIG. 4 illustrates a frontal view of the first exemplary embodiment of the surgical clip of FIGS. 1-3.
FIG. 5 illustrates a frontal view of a first leg member of the first exemplary embodiment of the surgical clip of FIGS. 1-4.
FIG. 6 illustrates a frontal view of a second leg member of the first exemplary embodiment of the surgical clip of FIGS. 1-5.
FIG. 7 illustrates a cut-away view of the first leg member of the first exemplary embodiment of the surgical clip of FIGS. 1-6.
FIG. 8 illustrates a cross-section of the cut-away view of FIG. 7.
FIG. 9 illustrates a cut-away away of the second leg member of the first exemplary embodiment of the surgical clip of FIGS. 1-8.
FIG. 10 illustrates a cross-section of the cut-away view of FIG. 9.
FIG. 11 illustrates a side view of a second exemplary embodiment of a surgical clip of the present invention.
FIG. 12 illustrates a side view of a compressed configuration of the second exemplary embodiment of the surgical clip of FIG. 11.
FIG. 13 illustrates a perspective view of the second exemplary embodiment of the surgical clip of FIGS. 11 and 12.
FIG. 14 illustrates a frontal view of the second exemplary embodiment of the surgical clip of FIGS. 11-13.
FIG. 15 illustrates a frontal view of a first leg member of the second exemplary embodiment of the surgical clip of FIGS. 11-14.
FIG. 16 illustrates a frontal view of a second leg member of the second exemplary embodiment of the surgical clip of FIGS. 11-15.

### Detailed Description

The invention will now be described with reference to the figures, in which like reference numerals may refer to like parts throughout. The present invention is generally directed to a surgical clip configured to compress and/or ligate tissue (*e.g.*, blood vessels, lymph nodes, nerves, cystic tubes, or cardiac tissue). The surgical clip may provide elongated leg members to increase the tissue-retaining capacity. For example, the surgical clip may be sized to fit in a 5 mm clip applier, but have an increased capacity compared to other 5 mm clips. To reinforce and stabilize the longer leg members, the surgical clip may have a wing member extending along at least one of the lateral sides of the leg members. The wing members may provide each of the leg members an increased aspect ratio (width/thickness). Thus, the surgical clip is relatively wider to increase the stiffness over the length of the leg members. The wing members may extend along the upper and lower periphery of the surgical clip, so the wing members do not interfere with the inner tissue ligation surfaces. The width may extend continuously through a hinge member to resist twisting and maintain alignment of the leg members. Furthermore, the width of the surgical clip at the wing members may be the same as the width of the surgical clip at the boss members, allowing the clip to lie flat on its side and improves feeding characteristics in automatic appliers. For example, the channel of an automatic clip applier may ensure lateral alignment of the surgical clip though contact with the side surfaces of the wing members, the boss members, and/or the hinge member. The automatic clip applier would therefore not need a separate alignment feature for the surgical clip. The width further protects against surgeons cutting the ligated vessel too close to the clip, which can compromise its retention on the vessel.

Additional aspects of the invention include improved teeth that receive a rail therebetween for increased axial security of tissue. The surgical clip may also have features that retain tissue proximate the hinge as the surgical clip closes and/or further stabilize and prevent rotating or inverting of the surgical clip. For example, in a first embodiment, the surgical clip includes convex surfaces pivotally attached to each of the tissue engaging surfaces. The convex surfaces may each include a tooth, and pinch and pull the tissue proximate the hinge member when the surgical clip closes. In a second embodiment, the surgical clip may include inner members extending from a proximal portion of each of the leg members. The inner members may be on opposing leg members and on opposing sides of the surgical clip. Each of the inner members may be aligned with an aperture through the opposing leg member. When the surgical clip closes the inner members may force the tissue into the opposing aperture. Furthermore, when the inner members are received in the aperture, the inner members may resist the surgical clip from inverting and/or rotating. However, the inner members may not latch and/or interlock to maintain flexibility of the leg members along their lengths.

In accordance with conventional practice, as used herein, and unless otherwise indicated herein, the term "proximal portion" refers to the specified portion of a device or its component which is generally closer to the medical personnel handling or manipulating the device as it is intended to be used, and the term "distal portion" refers to the specified portion of a device or its component which is opposite the proximal portion. The term "longitudinal" is directed to the dimension which extends along the length of the surgical clip and/or leg members from their respective proximal end portions to their respective distal end portions, as would be commonly understood by one of skill in the art. Furthermore, as used herein, the "transverse" direction is directed to any axis or direction which is orthogonal to the longitudinal lengths of the surgical clip or leg members. Accordingly, the term "length" refers to a dimension of the surgical clip and/or one or more components along its longitudinal direction. The term "vertical" refers to a dimension of the surgical clip and/or one or more components along a compression axis of the leg members. The term "thickness" refers to the dimension between opposing edges of the surgical clip and/or one or more components along the compression or vertical direction. The term "width" refers to a dimension of the surgical clip and/or one or more components in a lateral direction substantially transverse to the length and the thickness. The term "concave" and "convex" refers to the curvature of a surface or component visible when viewing an exterior of the surface or component. Similar terminology is used throughout the written disclosure.

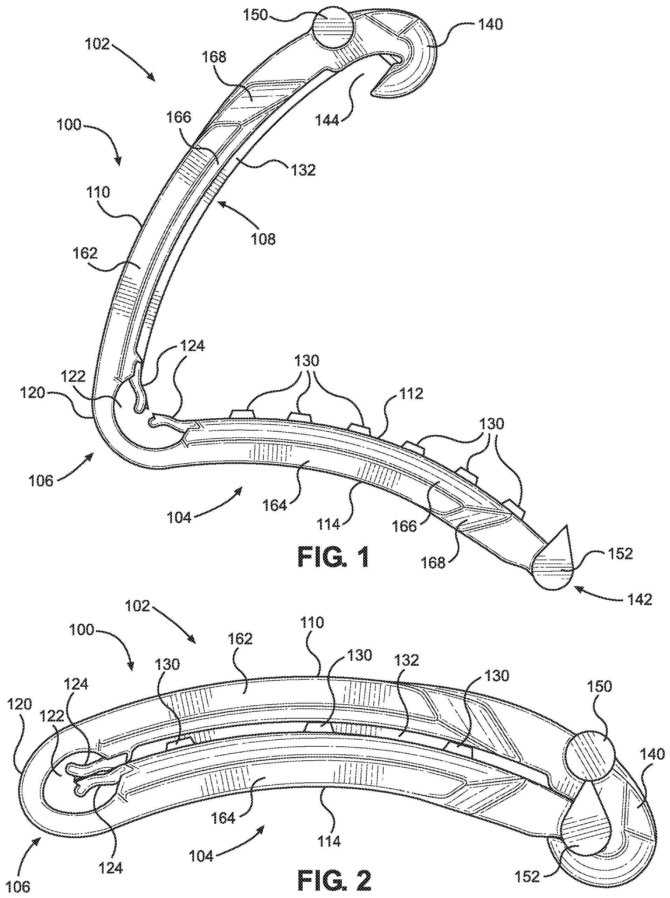

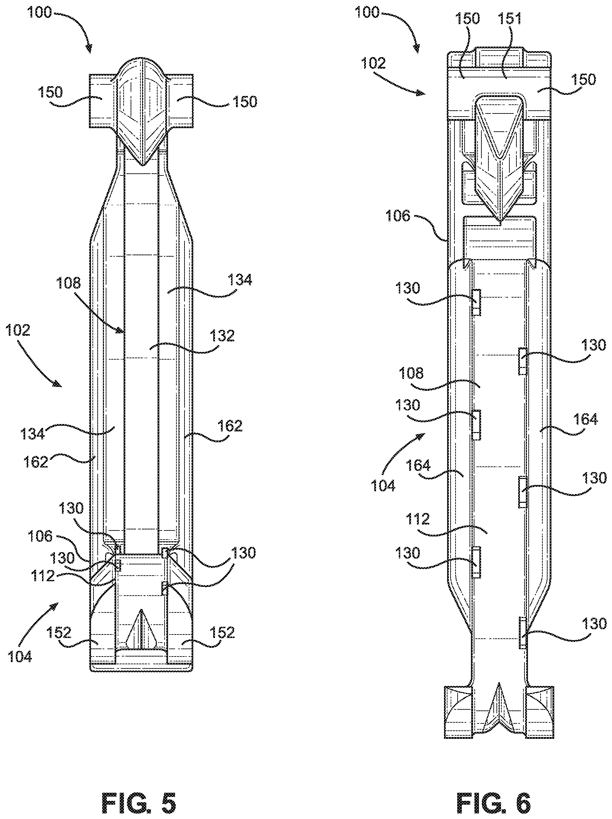

FIGS. 1-8 illustrate a first embodiment of a surgical clip 100 of the present invention. The surgical clip 100 may have a proximal end portion and a distal end portion. The surgical clip 100 may further include a first leg member 102 having a proximal end portion and a distal end portion, and a second leg member 104 having a proximal end portion and a distal end portion. The first and second leg members 102, 104 may be integrally joined at the proximal end portions by a hinge member 106.

The first and second leg members 102, 104 may include curved surfaces. For example, the first leg member 102 may include a first inner surface 108 and a first outer surface 110, and the second leg member 104 may include a second inner surface 112 and a second outer surface 114. As shown in FIG. 1, the first inner surface 108 may have a concave curvature, and the first outer surface 110 may have a convex curvature. The second inner surface 112 may have a convex curvature, and the second outer surface 114 may have a concave curvature. The curvatures of the first leg member 102 and the second leg member 104 may substantially match and the respective concavity/convexity of the first inner surface 108 and the second inner surface 112 may substantially match. The concave curvature of the first inner surface 108 and/or the convex curvature of the first outer surface 110 may extend substantially the entire length of the first leg member 102. The convex curvature of the second inner surface 112 and/or the concave curvature of the second outer surface 114 may extend substantially the entire length of the second leg member 104. The first and second inner surfaces 108, 112 may be approximated or contact in a closed configuration. Further discussion of the general curvatures of the leg members 102, 104 can be found in U.S. Patent No. 4,834,096, the entire disclosure of which is incorporated herein by reference.

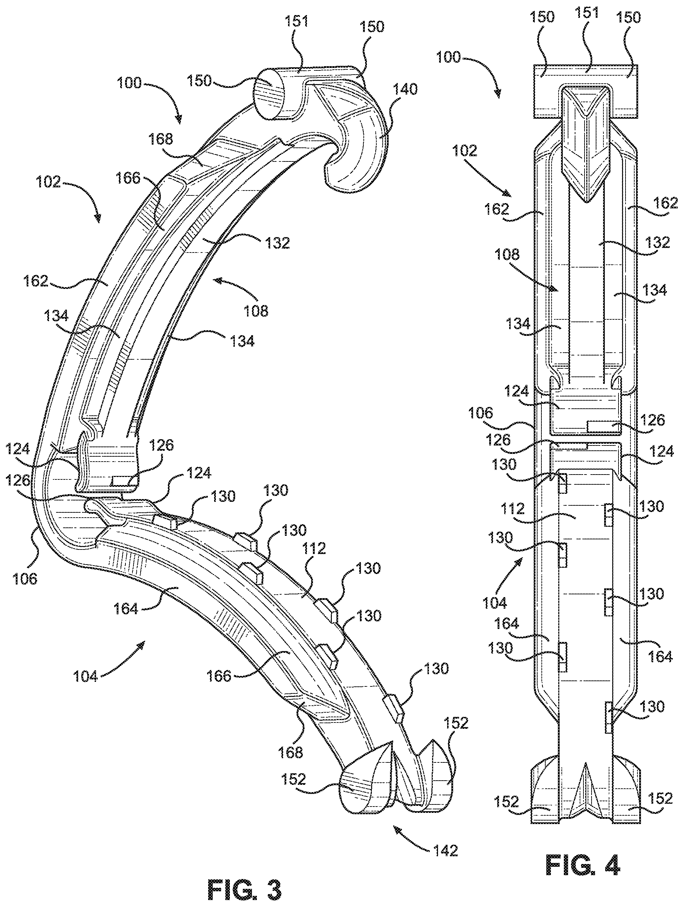

The hinge member 106 may be resiliently flexible and integral to the first and second leg members 102, 104. The hinge member 106 may have a convex outer surface 120 joining the first outer surface 110 and the second outer surface 114. The hinge member 106 may also include a slot 122 at least partially defined by the convex outer surface 120. The slot 122 may have a distal opening in the open configuration (*e.g.*, FIG. 1) to receive tissue and enable tissue retention. For example, the distal opening of the slot 122 may be defined by proximal flaps or extensions 124 extending from the first and second leg members 102, 104. The proximal extensions 124 may extend in a cantilever configuration allowing for pivoting and deflection relative to the first and second leg members 102, 104 based on the presence of tissue. The proximal extensions 124 may have convex inner surfaces and at least one tooth 126 configured to retain the tissue therebetween and provide a pinch-and-pull feature during closure. The proximal extensions 124 may extend proximally into the slot 122 (as exemplarily illustrated in FIG. 2) and be approximated or contact in the closed configuration. Thus, the convex inner surfaces of the proximal extensions 124 may be configured to securely retain tissue therebetween with the tissue extending into the slot 122. The teeth 126 may be laterally disposed and non-overlapping, and each proximal extension 124 may include a single laterally extending tooth 126 as exemplarily illustrated in FIGS. 3-4.

As further shown in the embodiment of FIGS. 1-6, the surgical clip 100 may include one or more teeth 130 on the inner surface 112 of the second leg member 104. The teeth 130 may be substantially rigid, such that the teeth 130 do not substantially deflect when engaging tissue. As illustrated in FIGS. 3-4, the teeth 130 may be spaced apart in first and second rows extending longitudinally on the inner surface 112 of the second leg member 102. The first and second rows of teeth 130 may be transversely separated from each other on opposite sides of a centerline of the inner surface 112 of the second curved leg member 104 on the periphery of the second inner surface 112. The teeth 130 of each the first and second rows may be staggered along the longitudinal axis of the inner surface 112. Correspondingly, the first inner surface 108 may define a rail 132 extending longitudinally on the first inner surface 108 and first and second longitudinal channels 134 extending on opposing lateral sides of the rail 132. Thus, in the closed configuration of the surgical clip 100 (*e.g.*, FIG. 2), the first row of teeth 130 may be received in the first channel 134, the second row of teeth 130 may be received in the second channel 134, and the rail 132 may be received between the first and second rows of teeth 130. The first and second longitudinal channels 134 may be substantially L-shaped and open on the lateral sides of the surgical clip 100. This configuration of the teeth 130 and rail 132 provides a favorable tortuous engagement of tissue with closely approximated tissue engaging surfaces. The teeth 130 may be larger and atraumatic, having a substantially flat tissue engaging inner surface. The larger, atraumatic teeth 130 may further improve tissue retention and prevent the tissue from slipping out of the surgical clip. However, in alternative embodiments, the teeth 130 may be on the first inner surface 108, and the rail 132 and channels 134 may be on the second inner surface 112.

The surgical clip 100 may also include a latching mechanism having one or more latching elements. For example, the first leg member 102 may transition to a hook member 140 at its distal end portion, and the second leg member 104 may transition to a complementary grooved and pointed tip member 142 at its distal end portion. A distal end portion of the hook member 140 may curve inwardly and point generally toward the hinge member 106. The hook member 140 may have one or more transverse beveled surfaces and a concave inner surface which merges with the first inner surface 108 to define a latching recess 144. The tip member 142 may be V-shaped defining a slot configured to receive the beveled surfaces of the hook member 140, as the hook member 140 deflects around the tip member 142 and/or the second leg member 104 compresses. The hook member 140 and the tip member 142 may engage to form the latching mechanism. For example, the latching recess 144 may receive the tip member 142 in the course of compressing the surgical clip 100 into the closed configuration (*e.g.*, FIG. 2) when secured position around a vessel or other tissue. Further discussion of the latching mechanism can be found in U.S. Patent No. 4,834,096, the entire disclosure of which is incorporated herein by reference.

The leg members 102, 104 may include one or more boss members along the length to engage jaws of the clip applier. For example, the first leg member 102 may include one or more boss members 150 protruding perpendicular to opposing side surfaces adjacent to the distal end portion of the first leg member 102 and immediately inward of the hook member 140. In the illustrated example of the surgical clip 100, the one or more boss members 150 may be cylindrical and project outwardly beyond the side surfaces of first leg member 102. The one or more boss members 150 may include a bridge section 151 extending the width of the first leg member 102. The second leg member 104 may also include one or more boss members 152 at the distal end portion. The boss members 152 may be cylindrical and protrude perpendicularly to opposing side surfaces of the second leg member 104, extending longitudinally forward beyond the point of tip member 142 and outwardly beyond the side surfaces of second leg member 104. The jaws of the clip applier may engage the boss members 150, 151, 152 and pivot the leg members 102, 104 about the hinge member 106 to compress the surgical clip 100 into a closed and/or latched configuration around a vessel.

The first leg member 102 may have at one first wing member 162 extending laterally of the first inner surface 108, and/or the second leg member 104 may have at least one second wing member 164 extending laterally of the second inner surface 112. For example, the at least one first wing member 162 may include a pair of first wing members 162 extending on opposing lateral sides of the first inner surface 108. The at least one second wing member 164 may include a pair of first wing members 164 extending on opposing lateral sides of the second inner surface 112. The wing members 162, 164 may reinforce and stabilize the leg members 102, 104 by increasing torsional stiffness. For example, the wing members 162, 164 may enable the leg members 162, 164 to be longer without compromising the torsional and/or tissue retention strength. The wing members 162, 164 may extend longitudinally for at least half of the length of the leg members 102, 104. In some embodiments, the wing members 162, 164 may extend longitudinally for at least two-thirds of the length of the leg members 102, 104. The wing members 162, 164 may extend continuously through the hinge member 106 to resist twisting of the hinge member 106 and maintain alignment of the leg members 102, 104, such that the width of the surgical clip 100 at the wing members 162, 164 may be substantially the same as a width of the surgical clip 100 at the hinge member 106. Furthermore, the width of the surgical clip 100 at the wing members 162, 164 may be the same as the width of the surgical clip 100 at the boss members 150, 151, 152, allowing the surgical clip 100 to lay flat on its side and improves feeding characteristics in automatic appliers. However, the wing members 162, 164 may have distal portions 168 spaced proximally from a distal end of the leg members 102, 104 and/or the boss members 150, 151, 152, such that the wing members 162, 164 do not extend the entire length of the leg members 102, 104 or interfere with the clip applier interfaces. For example, the distal portions 168 may be angled and/or beveled. The wing members 162, 164 may each have an inner surface 166 that are angled and/or beveled to provide a reduced thickness, such that the wing members 162, 164 do not interfere with tissue ligation.

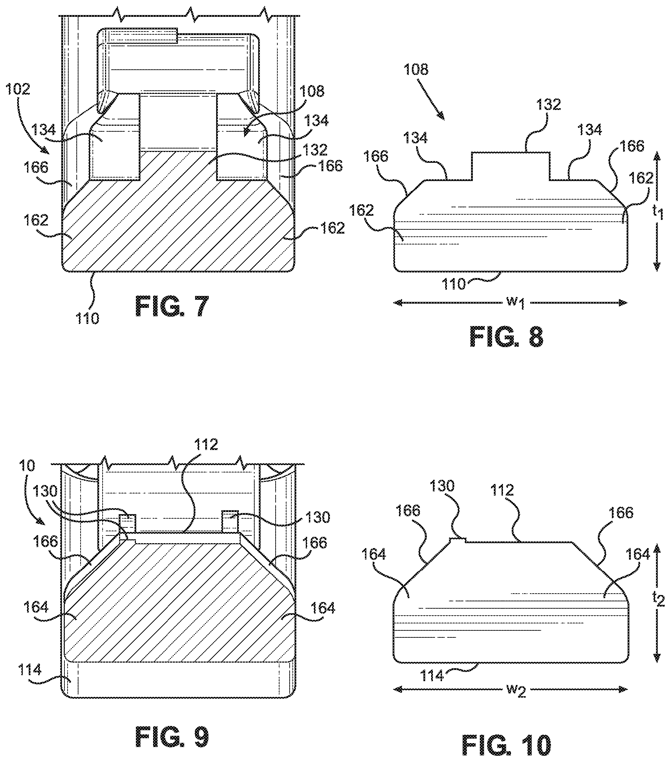

As further illustrated in the cut-away view of FIG. 7 and the cross-section of FIG. 8, the first leg member 102 may have a first width (w₁) greater than a first thickness (t₁). As similarly illustrated in the cut-away view of FIG. 9 and the cross-section of FIG. 10, the second leg member 104 may have a second width (w₂) greater than a second thickness (t₂). The thicknesses (t) may be defined by the distance between the respective inner surface 108, 112 and outer surface 110, 114. For example, the first thickness (t₁) may be defined by the distance between the inner surface of the rail 132 and the first outer surface 110. The second thickness (t₂) may be defined by the distance between the second inner surface 112 (excluding the teeth 130) and the second outer surface 114. The widths (w) may be defined by the distance between opposing side surfaces of the wing members 162, 164. The first width (w₁) may be at least 1.5 times greater than the first thickness (t₁), and/or the second width (w₂) may be at least 1.5 times greater than the second thickness (t₂). In some embodiments, the first width (w₁) may be about 2 times greater than the first thickness (t₁), and/or second width (w₂) may be about 2 times greater than the second thickness (t₂). The first width (w₁) and the second width (w₂) may be substantially the same, and/or the first thickness (t₁) and the second thickness (t₂) may be substantially the same. The first wing member 162 may have a thickness less than the first thickness (t₁), and/or the second wing member 164 may have a thickness less than the second thickness (t₂) to prevent interference with tissue ligation, for example due to the inner surfaces 166 having a bevel. The first wing member 162 may have an outer surface that is continuous or aligned with the first outer surface 110, and/or the second wing member 164 may have an outer surface that is continuous or aligned with the second outer surface 110. Each of the first wing members 162 may have a width no greater than a quarter of the first width (w₁), and/or each of the second wing members 164 may have a width no greater than a quarter of the second width (w₂). The side surface of each of the wing members 162, 164 may be substantially flat to provide a guide surface to prevent surgeons from cutting a ligated vessel too close to the surgical clip 100, which can compromise the retention of the surgical clip 100 on the vessel.

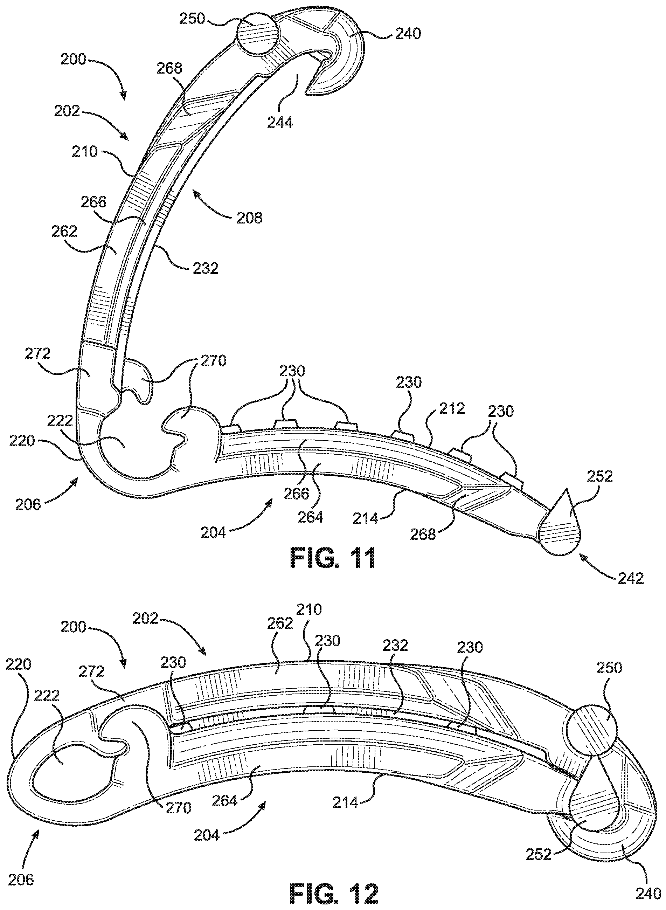

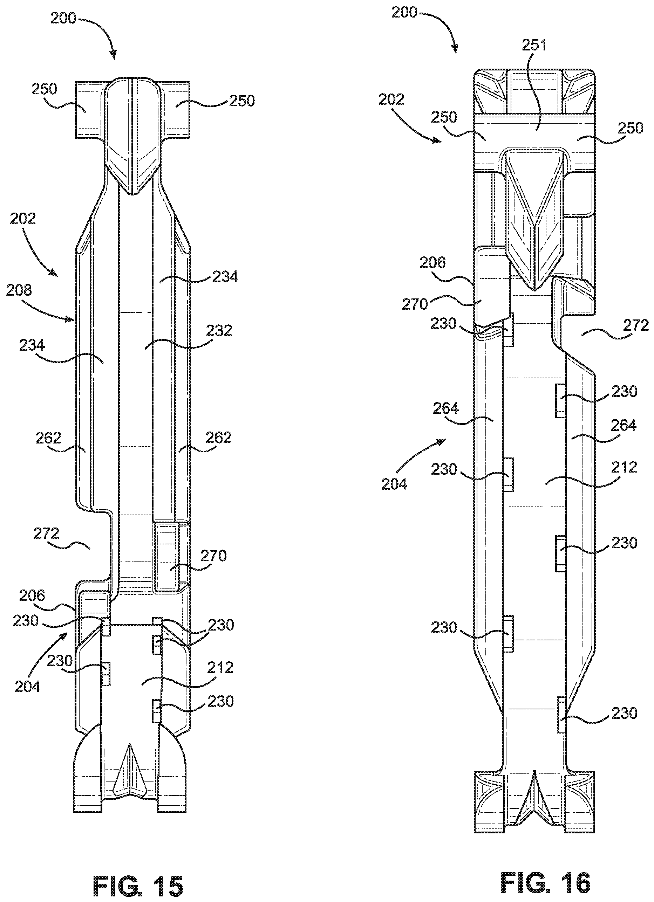

FIGS. 11-16 illustrate a second embodiment of a surgical clip 200 of the present invention. The surgical clip 200 may have a proximal end portion and a distal end portion. The surgical clip 200 may further include a first leg member 202 having a proximal end portion and a distal end portion, and a second leg member 204 having a proximal end portion and a distal end portion. The first and second leg members 202, 204 may be integrally joined at the proximal end portions by a hinge member 206. The surgical clip 200 may have similar features as the surgical clip 100 of the first embodiment including the cross-sections of FIGS. 7-10. Thus, the discussion of FIGS. 7-10 is incorporated herein with regard to the second embodiment, for the sake of brevity.

The first and second leg members 202, 204 may include curved surfaces. For example, the first leg member 202 may include a first inner surface 208 and a first outer surface 210, and the second leg member 204 may include a second inner surface 212 and a second outer surface 214. As shown in FIG. 11, the first inner surface 208 may have a concave curvature, and the first outer surface 210 may have a convex curvature. The second inner surface 212 may have a convex curvature, and the second outer surface 214 may have a concave curvature. The curvatures of the first leg member 202 and the second leg member 204 may substantially match, and the respective concavity/convexity of the first inner surface 208 and the second inner surface 212 may substantially match. The concave curvature of the first inner surface 208 and/or the convex curvature of the first outer surface 210 may extend substantially the entire length of the first leg member 202. The convex curvature of the second inner surface 212 and/or the convex curvature of the second outer surface 214 may extend substantially the entire length of the second leg member 04. The first and second inner surfaces 208, 212 may be approximated or contact in a closed configuration. Further discussion of the general curvatures of the leg members 202, 204 can be found in U.S. Patent No. 4,834,096, the entire disclosure of which is incorporated herein by reference.

The hinge member 206 may be resiliently flexible and integral to the first and second leg members 202, 204. The hinge member 206 may have a convex outer surface 220 joining the first outer surface 210 and the second outer surface 214. The hinge member 206 may also include a slot 222 at least partially defined by the convex outer surface 220. The slot 222 may have a distal opening in the open configuration (*e.g.*, FIG. 11) to receive tissue and enable tissue retention.

The first leg member may include a first inner member 270 and a first aperture 272 on a proximal portion, and the second leg member may include a second inner member 270 and a second aperture 272 on a proximal portion. The inner members 270 and apertures 272 may be on opposing sides of a centerline of the first and second leg members 202, 204 in a reflectively symmetric configuration, such that, in a closed configuration, the first aperture 272 receives the second inner member 270 and the second aperture 272 receives the first inner member 270. Thus, the inner members 270 and apertures 272 resist the surgical clip from inverting or laterally rotating in the closed configuration. However, the inner members 270 and apertures 272 may not latch and/or interlock to not inhibit the proximal portions of the leg members 202, 204 from flexing. The inner members 270 and apertures 272 may additionally increase the axially pull-off strength of the surgical clip 200. For example, the inner members 270 may force tissue into the opposing apertures 272 to pinch the the tissue therebetween. The inner members 270 may have a hook shape, and the apertures 272 may have open lateral sides.

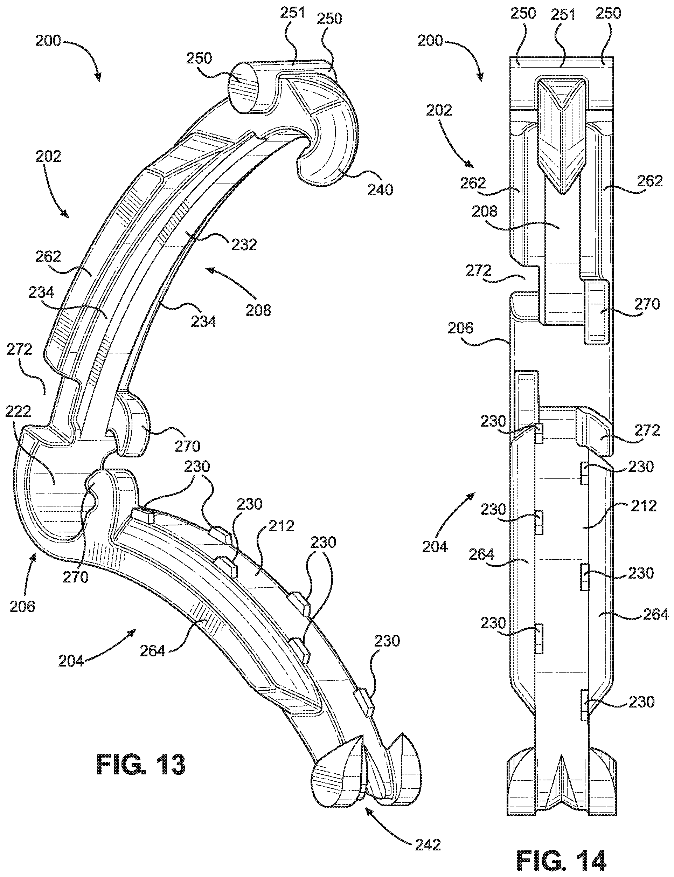

As further shown in the embodiment of FIGS. 11-16, the surgical clip 200 may include one or more teeth 230 on the inner surface 212 of the second leg member 204. The teeth 230 may be substantially rigid, such that the teeth 230 do not substantially deflect when engaging tissue. As illustrated in FIGS. 13-14, the teeth 230 may be spaced apart in first and second rows extending longitudinally on the inner surface 212 of the second leg member 202. The first and second rows of teeth 230 may be transversely separated from each other on opposite sides of a centerline of the inner surface 212 of the second leg member 204 on the periphery of the second inner surface 212. The teeth 230 of each the first and second rows may be staggered along the longitudinal axis of the inner surface 212. Correspondingly, the first inner surface 208 may define a rail 232 extending longitudinally on the first inner surface 208 and first and second longitudinal channels 234 extending on opposing lateral sides of the rail 232. Thus, in the closed configuration of the surgical clip 200 (*e.g.*, FIG. 12), the first row of teeth 230 may be received in the first channel 234, the second row of teeth 230 may be received in the second channel 234, and the rail 232 may be received between the first and second rows of teeth 230. The first and second longitudinal channels 234 may be substantially L-shaped and open on the lateral sides of the surgical clip 200. This configuration of the teeth 230 and rail 232 provides a favorable tortuous engagement of tissue with closely approximated tissue engaging surfaces. The teeth 230 may be larger and atraumatic, having a substantially flat tissue engaging inner surface. The larger, atraumatic teeth 230 may further improve tissue retention and prevent the tissue from slipping out of the surgical clip. However, in alternative embodiments, the teeth 230 may be on the first inner surface 208, and the rail 232 and channels 234 may be on the second inner surface 210.

The surgical clip 200 may also include a latching mechanism having one or more latching elements. For example, the first leg member 202 may transition to a hook member 240 at its distal end portion, and the second leg member 204 may transition to a complementary grooved and pointed tip member 242 at its distal end portion. A distal end portion of the hook member 240 may curve inwardly and point generally toward the hinge member 206. The hook member 240 may have one or more transverse beveled surfaces and a concave inner surface which merges with the first inner surface 208 to define a latching recess 244. The tip member 242 may be V-shaped defining a slot configured to receive the beveled surfaces of the hook member 240, as the hook member 240 deflects around the tip member 242 and/or the second leg member 204 compresses. The hook member 240 and the tip member 242 may engage to form the latching mechanism. For example, the latching recess 244 may receive the tip member 242 in the course of compressing the surgical clip 200 into the closed configuration (*e.g.*, FIG. 12) when secured position around a vessel or other tissue. Further discussion of the latching mechanism can be found in U.S. Patent No. 4,834,096, the entire disclosure of which is incorporated herein by reference.

The leg members 202, 204 may include one or more boss members along the length to engage jaws of the clip applier. For example, the first leg member 202 may include one or more boss members 250 protruding perpendicular to opposing side surfaces adjacent to the distal end portion of the first leg member 202 and immediately inward of the hook member 240. In the illustrated example of the surgical clip 200, the one or more boss members 250 may be cylindrical and project outwardly beyond the side surfaces of first leg member 202. The one or more boss members 250 may include a bridge section 251 extending the width of the first leg member 202. The second leg member 204 may also include one or more boss members 252 at the distal end portion. The boss members 252 may be cylindrical and protrude perpendicularly to opposing side surfaces of the second leg member 204, extending longitudinally forward beyond the point of tip member 242 and outwardly beyond the side surfaces of second leg member 204. The jaws of the clip applier may engage the boss members 250, 25, 252 and pivot the leg members 202, 204 about the hinge member 206 to compress the surgical clip 200 into a closed and/or latched configuration around a vessel.

The first leg member 202 may have at one first wing member 262 extending laterally of the first inner surface 208, and/or the second leg member 204 may have at least one second wing member 264 extending laterally of the second inner surface 212. For example, the at least one first wing member 262 may include a pair of first wing members 262 extending on opposing lateral sides of the first inner surface 208. The at least one second wing member 264 may include a pair of first wing members 264 extending on opposing lateral sides of the second inner surface 210. The wing members 262, 264 may reinforce and stabilize the leg members 202, 204 by increasing torsional stiffness. For example, the wing members 262, 264 may enable the leg members 262, 264 to be longer without compromising the torsional and/or tissue retention strength. The wing members 262, 264 may extend longitudinally for at least half of the length of the leg members 202, 204. In some embodiments, the wing members 262, 264 may extend longitudinally for at least two-thirds of the length of the leg members 202, 204. The wing members 262, 264 may extend continuously through the hinge member 206 to resist twisting of the hinge member 206 and maintain alignment of the leg members 202, 204, such that the width of the surgical clip 200 at the wing members 262, 264 may be substantially the same as a width of the surgical clip 200 at the hinge member 206. Furthermore, the width of the surgical clip 200 at the wing members 262, 264 may be the same as the width of the surgical clip 200 at the boss members 250, 251, 252, allowing the surgical clip 200 to lay flat on its side and improves feeding characteristics in automatic appliers. However, the wing members 262, 264 may have distal portions 268 spaced proximally from the boss members 250, 251, 252, such that the wing members 262, 264 do not interfere with the clip applier interfaces. For example, the distal portions 268 may be angled and/or beveled. The wing members 262, 264 may each have an inner surface 266 that are angled and/or beveled to provide a reduced thickness, such that the wing members 262, 264 do not interfere with tissue ligation.

The various embodiments of the surgical clips 100, 200 of the present invention may be made of any suitable size and may be applied to any number of tissues, such as blood vessels, lymph nodes, nerves, cystic ducts, and cardiac tissue. The various embodiments of the surgical clips 100, 200 may be constructed from any suitable biocompatible material, such as metals and polymers. However, the present invention is particularly suitable for practice with polymeric clips. Thus, the various embodiments of the surgical clips 100, 200 preferably consist of a one-piece integral polymeric body formed from a suitable strong biocompatible engineering plastic such as the type commonly used for surgical implants. Exemplary materials include homopolymer or co-polymer polyacetal, polyethylene terephthalate (PET), polybutylene terephthalate (PBT), polyoxymethylene, or other thermoplastic materials having similar properties that can be injection-molded, extruded, or otherwise processed into like articles.
Some further aspects of the invention can be found in the following paragraphs:
1. A surgical clip comprising:
   a first leg member having a first inner surface with a concave curvature, the first leg member having a first thickness in a compression direction, a first width in a lateral direction, and a first length in a longitudinal direction;
   a second leg member having a second inner surface with a convex curvature, the second leg member having a second thickness in the compression direction, a second width in the lateral direction, and a first length in the longitudinal direction; and
   a hinge member pivotally coupling the first leg member and the second leg member,
   wherein the first width is greater than the first thickness along at least half of the first length, and/or the second width is greater than the second thickness along at least half of the second length.
2. The surgical clip of paragraph 1, wherein the first width is at least 1.5 times greater than the first thickness and/or the second width is at least 1.5 times greater than the second thickness.
3. The surgical clip of paragraph 2, wherein the first width is about 2 times greater than the first thickness and/or the second width is about 2 times greater than the second thickness.
4. The surgical clip of at least one of paragraphs 1-3, wherein the first width is greater than the first thickness along at least about two-thirds of the first length, and/or the second width is greater than the second thickness along at least about two-thirds of the second length.
5. The surgical clip of at least one of paragraphs 1-4, further comprising:
   at least one first boss member on the first leg member; and
   at least one second boss member on the second leg member,
   wherein the first width is substantially the same as a width of the surgical clip at the at least one first boss member, and/or the second width is substantially the same as a width of the surgical clip at the at least one second boss member.
6. The surgical clip of at least one of paragraphs 1-5, wherein the first width is substantially the same as a width of the hinge member, and/or the second width is substantially the same as the width of the hinge member.
7. The surgical clip of at least one of paragraphs 1-6, wherein the first leg member includes at least one first wing member extending laterally of the first inner surface to define the first width, and/or the second leg member includes at least one second wing member extending laterally of the second inner surface to define the second width.
8. The surgical clip of paragraph 7, wherein the at least one first wing member includes a pair of first wing members, and/or the at least one second wing member includes a pair of second wing members.
9. The surgical clip of at least one of paragraphs 7-8, wherein a distal portion of the at least one first wing member is spaced proximally from a distal end of the first leg member and/or a distal portion of the at least one second wing member is spaced proximally from a distal end of the second leg member.
10. The surgical clip of at least one of paragraphs 7-9, wherein the at least one first wing member has a thickness less than the first thickness and/or the at least one second wing member has a thickness less than the second thickness.
11. The surgical clip of paragraphs 7-10, wherein the at least one first wing member and/or the at least one second wing member includes an inner surface that is beveled.
12. The surgical clip of at least one of paragraphs 7-11, wherein the at least one first wing member includes an outer surface that is continuous with an outer surface of the first leg member and/or the at least one second wing member includes an outer surface that is continuous with an outer surface of the second leg member.
13. The surgical clip of at least one of paragraphs 7-12, wherein each of the at least one first wing member has a width no great than about a quarter of the first width and/or each of the at least one second wing member has a width no greater than about a quarter of the second width.
14. The surgical clip of at least one of paragraphs 7-13, wherein the at least one first wing member and/or the at least one second wing member includes a side surface that is substantially flat.
15. The surgical clip of at least one of paragraphs 1-14, further comprising:
   a hook member on a distal portion of the first leg member; and
   a tip member on a distal portion of the second leg member,
   wherein the hook member is configured to receive the tip member to retain the surgical clip in a closed configuration.
16. The surgical clip of at least one of paragraphs 1-15, further comprising:
   first and second rows of teeth extending from one of the first inner surface and the second inner surface;
   first and second channels on another of the first inner surface and the second inner surface;
   wherein, in a closed configuration, the first channel is configured to receive the first row of teeth, and the second channel is configured to receive the second row of teeth.
17. The surgical clip of at least one of paragraphs 1-16, further comprising a first proximal extension extending from the first leg member toward the hinge member; and a second proximal extension extending from the second leg member toward the hinge member.
18. The surgical clip of at least one of paragraphs 1-17, wherein the first proximal extension comprises an inner surface having a convex curvature, and the second proximal extension comprises an inner surface having a convex curvature.
19. The surgical clip of a least one of paragraphs 1-18, further comprising:
   a first inner member at a proximal portion of the first leg member; and
   a first aperture at a proximal portion of the second leg member,
   wherein the first aperture is configured to receive the first inner member in a closed configuration to resist the surgical clip from inverting and/or rotating, and the first inner member does not latch and/or interlock with the first aperture.
20. The surgical clip of a least one of paragraphs 1-19 further comprising:
   a second inner member at a proximal portion of the second leg member; and
   a second aperture at a proximal portion of the first leg member,
   wherein the second aperture is configured to receive the second inner member in a closed configuration to resist the surgical clip from inverting and/or rotating, and the second member does not latch and/or interlock with the second aperture.

## Claims

1. A surgical clip comprising:
a first leg member having a first inner surface with a first curvature, the first leg member having a first thickness in a compression direction, a first width in a lateral direction, and a first length in a longitudinal direction;
a second leg member having a second inner surface with a second curvature, the second leg member having a second thickness in the compression direction, a second width in the lateral direction, and a second length in the longitudinal direction; and
a hinge member pivotally coupling the first leg member and the second leg member;
wherein the first leg member includes at least one first wing member extending laterally of the first inner surface such that a distal portion of the at least one first wing member is spaced proximally from a distal end of the first leg member and/or the second leg member includes at least one second wing member extending laterally of the second inner surface such that a distal portion of the at least one second wing member is spaced proximally from a distal end of the second leg member.

2. The surgical clip of claim 1, wherein the first width is greater than the first thickness along at least half of the first length, and/or the second width is greater than the second thickness along at least half of the second length.

3. The surgical clip of any one of claims 1-2, further comprising at least one first boss member on the first leg member; and at least one second boss member on the second leg member, wherein the first width is substantially the same as a width of the surgical clip at the at least one first boss member, and/or the second width is substantially the same as a width of the surgical clip at the at least one second boss member.

4. The surgical clip of any one of claims 1-2, wherein the at least one first wing member includes a pair of first wing members, and/or the at least one second wing member includes a pair of second wing members.

5. The surgical clip of any one of claims 1-2, wherein the at least one first wing member has a thickness less than the first thickness, and/or the at least one second wing member has a thickness less than the second thickness.

6. The surgical clip of any one of claims 1-2, wherein the at least one first wing member and/or the at least one second wing member includes an inner surface that is beveled.

7. The surgical clip of any one of claims 1-2, wherein each of the at least one first wing member has a width no greater than about a quarter of the first width and/or each of the at least one second wing member has a width no greater than about a quarter of the second width.

8. The surgical clip of any one of claims 1-2, wherein the at least one first wing member and/or the at least one second wing member includes a side surface that is substantially flat.

9. The surgical clip of any one of claims 1-2, further comprising a hook member on a distal portion of the first leg member; and a tip member on a distal portion of the second leg member, wherein the hook member is configured to receive the tip member to retain the surgical clip in a closed configuration.

10. The surgical clip of any one of claims 1-2, further comprising first and second rows of teeth extending from one of the first inner surface and the second inner surface; first and second channels on another of the first inner surface and the second inner surface; wherein, in a closed configuration, the first channel is configured to receive the first row of teeth, and the second channel is configured to receive the second row of teeth.
